(19) Europäisches Patentamt
European Patent Office
Office européen des brevets

(11) **EP 3 023 082 B1**

(12) **EUROPÄISCHE PATENTSCHRIFT**

(45) Veröffentlichungstag und Bekanntmachung des Hinweises auf die Patenterteilung:
**06.05.2020 Patentblatt 2020/19**

(51) Int Cl.:
***A61F 9/008*** *(2006.01)*

(21) Anmeldenummer: **14003938.9**

(22) Anmeldetag: **24.11.2014**

(54) **Vorrichtung zum Schützen von Augengewebe bei Laserbehandlungen**

Device for protecting eye tissue during laser treatments

Dispositif de protection de tissus oculaires lors de traitements au laser

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**25.05.2016 Patentblatt 2016/21**

(73) Patentinhaber: **Ziemer Ophthalmic Systems AG**
**2562 Port (CH)**

(72) Erfinder: **Rathjen, Christian**
**28197 Bremen (DE)**

(74) Vertreter: **Rentsch Partner AG**
**Bellerivestrasse 203**
**Postfach**
**8034 Zürich (CH)**

(56) Entgegenhaltungen:
**EP-A1- 2 705 812     US-A1- 2008 033 408**

EP 3 023 082 B1

**Beschreibung**

Technisches Gebiet

Die vorliegende Erfindung betrifft eine Vorrichtung zum Schützen von Augengewebe bei Laserbehandlungen. Die vorliegende Erfindung betrifft insbesondere eine Vorrichtung zum Schützen von Gewebe bei der Behandlung eines Auges mit einem von einem optischen Therapiesystem erzeugten und durch ein Scannersystem abgelenkten Laserstrahl.

Stand der Technik

Bei der Behandlung von Augengewebe, beispielsweise zur refraktiven Korrektur der Hornhaut oder Linse, werden ophthalmologische Laserbehandlungssysteme eingesetzt, welche Laserstrahlung, insbesondere gepulste Laserstrahlung, fokussiert zu projizieren, z.B. auf gezielte Punkte oder entlang einer Bearbeitungslinie um so Gewebe zum Abtragen von Gewebeschichten oder zur Erzeugung von Gewebeschnitten aufzulösen. Vorbereitend wird die Geometrie und Topographie des zu behandelnden Auges und seiner Strukturen erfasst und die geplante Bearbeitung mittels Bearbeitungsdaten definiert. Da der Laserstrahl über den Fokus hinaus in Augengewebe eingestrahlt wird, besteht die Gefahr, dass unerwünschte Stellen im Auge durch Laserstrahlung beeinträchtigt werden und unbeabsichtigte, falsche Gewebebereiche oder Strukturen im Auge durch den Laserstrahl bearbeitet und/oder zu stark belastet werden, so dass Schäden durch photochemische, photothermische und/oder photoakustische Effekte entstehen. Um derartige Schäden zu vermeiden werden Grenzwerte für die Strahlenergie respektive Strahldosis festgelegt, die die Sicherheit des Augengewebes gewährleisten sollen. Gemäss US 2014/0276680 wird die Laserenergie auf einen Maximalwert begrenzt, um die Retina höchstens mit einer entsprechend einer Sicherheitsnorm begrenzten Spitzenlaserenergie zu bestrahlen, beispielsweise eine Sicherheitsnorm gemäss ANSI Z136.1-2000 oder ISO 15004. Dabei besteht allerdings das Problem, dass die tatsächliche verabreichte Strahldosis stark von der verwendeten Behandlungsprozedur und damit verbundenen Strahlparametern, z.B. Strahlleistungshöhe oder Numerische Apertur (NA), dynamische Veränderungen von Strahlparametern, Fokustiefe, dynamische Veränderung der Fokustiefe, Spotgrösse, dynamische Veränderung der Spotgrösse, und/oder vom Scannmuster abhängt, so dass die Bestimmung der lokalen Strahldosis und damit insbesondere der maximalen lokalen Strahldosis mit analytischen Methoden unmöglich ist, da sich keine hinreichend exakten Berechnungsverfahren herleiten lassen. Bei der Anwendung optischer Therapiesysteme mit scannenden Lasersystemen zur Kataraktbehandlung ändert sich beispielsweise der Durchmesser des von der Laserstrahlung erzeugten Leuchtflecks auf der Retina stark mit der Fokustiefe. Bei Therapie-Systemen für Schnitte in der Hornhaut bestehen derart starke Abhängigkeiten hingegen nicht. Somit sind zur Respektierung von Lasernormen in einem Worst Case Ansatz alle relevanten Parameter in ihrer negativsten Ausprägung anzunehmen. Dies schränkt die erreichbare Performance der Behandlungssysteme und -verfahren ein, wodurch beispielsweise die Behandlungszeit unnötig verlängert wird, weil die mittlere Leistung in einem Worst Case Ansatz beschränkt werden muss. Der Patient wird somit längeren Vakuumzeiten ausgesetzt und es besteht zusätzlich ein erhöhtes Risiko des Abbruchs der Behandlung, da ein Patient nur für eine begrenzte Zeitdauer still halten kann. Überdies ist es auch nicht möglich, die Definition von Schnitten respektive ganzer Behandlungsprozeduren durch den Benutzer durchführen zu verlassen, da dieser die Überprüfung der Einhaltung zulässiger (maximaler) Dosiswerte in der Regel nicht durchführen kann, da diese zu komplex ist.

EP 2705812 beschreibt eine Vorrichtung zum Laserschneiden innerhalb transparenter Materialien, welche eine Einrichtung zur Ermittlung der Bestrahlungsdosis pro Flächeneinheit umfasst. Gemäss EP 2705812 erfolgt die Berechnung der Bestrahlungsdosis pro Flächeneinheit aus Bestrahlungsparametern der Laserlichtquelle und vorbestimmten Spotpositionen, die durch auswählbare bestehende Spotraster definiert sind. Gemäss EP 2705812 hängt der wirksame Schneidemechanismus vom Abstand der Rasterpunkte und von der Energiedichte der Plasmen ab, wobei die Energiedichte der Plasmen bei ansonsten gleichen Bestrahlungsparametern durch die Laserpulsenergie einstellbar ist. Gemäss EP 2705812 ist die Berechnung der Bestrahlungsdosis pro Flächeneinheit somit auf eine Auflösung begrenzt, die durch das jeweilige Spotraster bestimmt ist. Über eine Datenschnittstelle zum Scanner und ggf. zur Laserlichtquelle stehen der Einrichtung die Bestrahlungsparameter und die auswählbaren Spotraster bereits vor der Bestrahlung zur Verfügung, und sie kann auf der Basis einer Vorausberechnung der Bestrahlungsdosis einen Dosiswert ausgeben oder Warnanzeigen aktivieren, wenn der errechnete Dosiswert einen vorbestimmten Schwellenwert übersteigt. Schliesslich beschreibt EP 2705812 eine Ausführungsvariante, in der bei voreingestellten Laserparametern sehr engmaschige Spotraster, die zu einer über den vorbestimmten Schwellenwert hinausgehenden Bestrahlungsdosis pro Flächeneinheit führen würden, gar nicht mehr auswählbar sind oder nur durch gesonderte Freigabe durch den Nutzer aktiviert werden können.

Darstellung der Erfindung

Es ist eine Aufgabe der vorliegenden Erfindung eine Vorrichtung zum Schützen von Augengewebe bei Laserbehandlungen vorzuschlagen, welche zumindest einige Nachteile der bekannten Systeme nicht aufweist. Es ist insbesondere eine Aufgabe der vorliegenden Erfindung eine Vorrichtung zum Schützen von Augengewebe bei Laserbehandlungen vorzuschlagen, welche eine sichere Einhaltung von Strahldosisgrenzwerten ermöglicht, ohne dazu sämtliche Systemparameter fest auf Sicherheitswerte für Worst Case Szenarien einstellen zu müssen.

Gemäss der vorliegenden Erfindung werden diese Ziele durch die Merkmale des unabhängigen Anspruchs erreicht. Weitere vorteilhafte Ausführungsformen gehen ausserdem aus den abhängigen Ansprüchen und der Beschreibung hervor.

Eine Vorrichtung zum Schützen von Gewebe bei der Behandlung eines Auges mit einem von einem optischen Therapiesystem erzeugten und durch ein Scannersystem abgelenkten Laserstrahl umfasst ein Augenmodell, das Augendaten umfasst, welche Abmessungen und Lage von Augenstrukturen definieren, sowie ein Steuerdatenmodul, das eingerichtet ist, Steuerdaten zu erfassen, welche Strahlparameter des Laserstrahls und ein Scannmuster für den Laserstrahl definieren.

Die oben genannten Ziele werden durch die vorliegende Erfindung insbesondere dadurch erreicht, dass die Vorrichtung zum Schützen von Gewebe überdies einen Prozessor umfasst, der eingerichtet ist, basierend auf den Augendaten und den Steuerdaten rechnerisch einen sich auf oder in einer Augenstruktur bewegenden Lichtfleck zu simulieren, für mehrere Messpunkte der Augenstruktur laufend mit dem sich bewegenden Lichtfleck eine am betreffenden Messpunkt durch den sich bewegenden Lichtfleck abgegebene Strahldosis aufzurechnen, und beim Überschreiten eines Dosisgrenzwerts $D_{max}$ an einem der Messpunkte ein Notsignal zu erzeugen.

Durch die Simulation des sich entsprechend der geplanten oder ausgeführten Laserbehandlung bewegenden Lichtflecks und die Akkumulierung der dabei eingestrahlten Laserenergie an konkreten Messpunkten im Auge wird eine realitätsnahe Bestimmung der Strahldosis mit hoher örtlicher Auflösung im Auge ermöglicht (auf und in Augenstrukturen und deren Oberflächen), was im Vergleich zu Worst Case Ansätzen, bei denen zur Einhaltung von Sicherheitsnormen Laser-, Augen-, und Behandlungsparameter in ihrer negativsten Ausprägung angenommen werden, den Einsatz höherer mittlerer Laserleistungen und damit einhergehende kürzere Behandlungszeiten bei gleichzeitiger Einhaltung von Sicherheitsnormen ermöglicht.

In einer Ausführungsvariante definieren die Steuerdaten eine Scanngeschwindigkeit für das optische Therapiesystem, und der Prozessor ist eingerichtet, den sich bewegenden Lichtfleck mit einer auf der Scanngeschwindigkeit basierenden Bewegungsgeschwindigkeit zu simulieren.

In einer Ausführungsvariante umfassen die Strahlparameter die momentane Strahlleistung des Laserstrahls, und der Prozessor ist eingerichtet, die Strahldosis an den Messpunkten jeweils als Energiewert durch Integration der Strahlleistung des Laserstrahls aus dem sich über den betreffenden Messpunkt bewegenden Lichtfleck aufzurechnen.

In einer Ausführungsvariante ist der Prozessor eingerichtet, für die Messpunkte der Augenstruktur laufend eine sich durch den bewegenden Lichtfleck ergebende Bestrahlungszeitdauer zu ermitteln, und den Dosisgrenzwert $D_{max}$ für die Messpunkte jeweils abhängig von der Bestrahlungszeitdauer am betreffenden Messpunkt zu definieren.

In einer Ausführungsvariante ist der Prozessor eingerichtet, den Dosisgrenzwert $D_{max}$ für die Messpunkte abhängig von der Wellenlänge oder den Wellenlängen des Laserstrahls zu definieren.

In einer Ausführungsvariante ist der Prozessor eingerichtet, den Dosisgrenzwert $D_{max}$ für die Messpunkte abhängig von einer Bestrahlungszeitdauer am betreffenden Messpunkt zu bestimmen.

In einer Ausführungsvariante ist der Prozessor eingerichtet, den Dosisgrenzwert $D_{max}$ für die Messpunkte jeweils gemäss der Formel $D_{max} = C \cdot t^{\frac{3}{4}}$ zu berechnen, wobei $C$ eine Strahlparametern (insbesondere Wellenlänge, Pulslänge/Pulsdauer und Divergenzwinkel) des Laserstrahls abhängige Konstante und $t$ die Bestrahlungszeitdauer am betreffenden Messpunkt sind.

In einer Ausführungsvariante ist der Prozessor eingerichtet, den abgelenkten Laserstrahl basierend auf dem durch die Steuerdaten definierten Scannmuster zu simulieren, die Augenstruktur basierend auf den Augendaten zu modellieren, und den sich bewegenden Lichtfleck basierend auf dem simulierten abgelenkten Laserstrahl und der modellierten Augenstruktur zu simulieren.

In einer Ausführungsvariante ist der Prozessor eingerichtet, den sich bewegenden Lichtfleck auf einer Oberfläche der Augenstruktur zu ermitteln, und die sich durch den bewegenden Lichtfleck ergebende Strahldosis für mehrere Messpunkte auf der Oberfläche der Augenstruktur aufzurechnen.

In einer Ausführungsvariante ist der Prozessor eingerichtet, den sich bewegenden Lichtfleck und die Messpunkte auf einer Oberfläche einer oder mehrerer Augenstrukturen aus der folgenden Liste zu bestimmen: Epithel, Endothel, Iris, Sklera, vordere Linsenoberfläche, hintere Linsenoberfläche und Netzhautoberfläche.

In einer Ausführungsvariante ist die Vorrichtung mit dem optischen Therapiesystem und dem Scannersystem verbunden, und der Prozessor ist eingerichtet, die Steuerdaten während der Behandlung des Auges vom optischen Therapiesystem und vom Scannersystem zu erfassen, und das Notsignal zum Unterbrechen der Behandlung an das optische Therapiesystem zu übermitteln.

In einer Ausführungsvariante ist der Prozessor eingerichtet, die Steuerdaten zur Simulation der Behandlung des Auges über eine Benutzerschnittstelle zu erfassen, und das Notsignal als Warnhinweis über die Benutzerschnittstelle auszugeben.

In einer Ausführungsvariante definieren die Steuerdaten einen oder mehrere Strahlparameter aus der folgenden Liste: mittlere Stahlleistung, Pulsbreite, Pulsrate, Pulsenergie, Pulsintensität, Fokusgrösse, Laserstrahlintensitätsprofil, und Divergenz des zur fokussierten Projektion vorgesehenen Laserstrahls, und der Prozessor ist eingerichtet, den sich bewegenden Lichtfleck basierend auf dem einen oder mehreren Strahlparametern zu simulieren.

In einer Ausführungsvariante umfasst die Vorrichtung überdies ein Messsystem, das eingerichtet ist, die Augendaten während der Behandlung des Auges zu bestimmen.

In einer Ausführungsvariante umfasst die Vorrichtung überdies ein Positionierungssystem, das eingerichtet ist, während der Behandlung eine Relativposition des Auges zu bestimmen und das Augenmodell abhängig von der Relativposition bezüglich des optischen Therapiesystems zu positionieren.

Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:

Figur 1a: zeigt schematisch im Querschnitt ein Auge unter Behandlung eines von einem optischen Therapiesystem erzeugten und durch ein Scannersystem abgelenkten Laserstrahls, sowie eine Vorrichtung zum Schützen von Gewebe bei der Behandlung des Auges.

Figur 1b: zeigt ein Intensitätsprofil im Strahlquerschnitt des Laserstrahls, und in der Aufsicht, ein dem Intensitätsprofil entsprechender Lichtfleck auf einem in der Nähe der Retina positionierten (virtuellen) Array.

Figur 2: zeigt ein Flussdiagramm, das beispielhaft eine Sequenz von Schritten zum Schützen von Gewebe bei der Behandlung eines Auges mit einem Laserstrahl illustriert.

Wege zur Ausführung der Erfindung

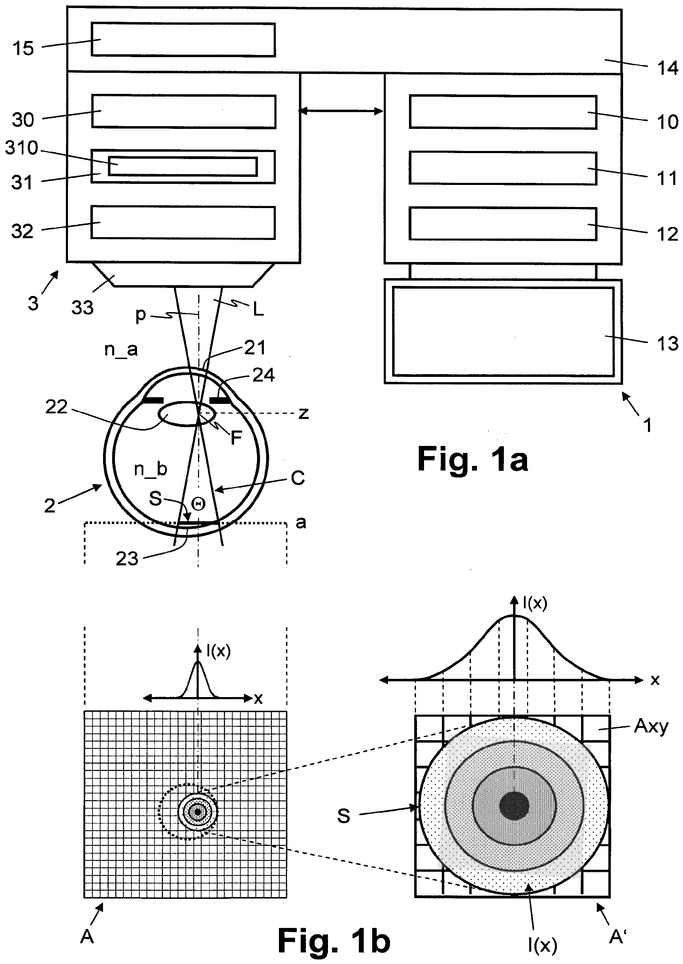

In der Figur 1a bezieht sich das Bezugszeichen 1 auf eine Vorrichtung zum Schützen von Gewebe bei der Behandlung eines Auges 2 mit einem von einem Lasersystem 31 eines optischen Therapiesystems 3 erzeugten und durch ein Scannersystem 32 abgelenkten Laserstrahl L. Je nach Ausführungsvariante ist die Schutzvorrichtung 1 mit dem optischen Therapiesystem 3 in einem gemeinsamen Gehäuse oder separat vom optischen Therapiesystem 3 in einem eigenen Gehäuse angeordnet.

Das optische Therapiesystem 3 umfasst neben dem Scannersystem 32 ein Lasersystem 31 mit einer Laserquelle 310 und eine Steuereinheit 30 zur Steuerung des Lasersystems 31, insbesondere zur Steuerung der Laserquelle 310 und des Scannersystems 32. Die Steuereinheit 30 umfasst einen programmierbaren Prozessor mit Daten- und Programmspeicher. Die Laserquelle 310 ist eingerichtet einen gepulsten Laserstrahl L, insbesondere mit Femtolaserpulsen, zu erzeugen. Dabei sind Laser- respektive Strahlparameter wie Laserleistung, Strahlleistung des Laserstrahls L, Pulsbreite, Pulsrate, Pulsenergie, Pulsintensität, und/oder Laserstrahlintensitätsprofil durch die Steuereinheit 30 einstellbar. Das Scannersystem 32 umfasst beispielsweise verschiebbare Optiken und/oder einen oder mehrere bewegliche (z.B. rotierbare) Spiegel zur Ablenkung des Laserstrahls L gemäss einem Scannmuster und mit einer Scanngeschwindigkeit, die durch die Steuereinheit 30 definierbar sind. Über die Steuereinheit 30 sind im Lasersystem 31 auch Fokusgrösse und Divergenz des zur fokussierten Projektion vorgesehenen Laserstrahls L einstellbar. Je nach Ausführungform kann das Scannersystem auch die Mittel zur Fokussierung und Strahlformung enthalten.

Die Schutzvorrichtung 1 umfasst einen Prozessor 10, ein Steuerdatenmodul 11 und ein Augenmodell 12. Der Prozessor 10 umfasst einen integrierten Schaltkreis, der beispielsweise als programmierbarer Mikroprozessor oder als andere programmierbare Logikeinheit, z.B. als ASIC (Application Specific Integrated Circuit), ausgeführt ist. Der Prozessor 10 umfasst einen Datenspeicher und ist derart eingerichtet respektive programmiert, dass er die später beschriebenen Funktionen zum Schützen von Augengewebe bei Laserbehandlungen ausführt.

Das Augenmodell 12 umfasst Augendaten, die Abmessungen, Form und Lage von Augenstrukturen des Auges 2 definieren, beispielsweise Cornea 21, Epithel, Endothel, Iris 24, Sklera, Linse 22, vordere Linsenoberfläche, hintere Linsenoberfläche und/oder Netzhautoberfläche 23 (Retina). In einer Ausführungsvariante sind die Augenstrukturen respektive Oberflächen der Augenstrukturen jeweils durch einen zweidimensionalen Array A modelliert, wobei die Array-Elemente Axy jeweils einem Flächenelement oder Pixel einer Augenstruktur respektive Oberfläche der Augestruktur mit einem zugeordneten Messpunkt der betreffenden Augenstruktur respektive der Oberfläche der Augenstruktur entsprechen. Die Flächenelemente respektive Pixel sind quadratisch und weisen eine Länge respektive Breite von 1 0µm-500µm auf. In seiner einfachsten Form entspricht der Array A den Zellen eines Gitternetzes, das auf einer horizontalen (normal zur Projektionsachse p verlaufenden) Ebene angeordnet ist, welche in Richtung der Projektionsachse p in der Nähe der betreffenden Augenstruktur positioniert ist, beispielsweise als Tangentialebene durch einen Apex der Augenstruktur, oder an einem Mittelpunkt oder Schwerpunkt der Augenstruktur entlang einer Zentrumsachse in Richtung der Projektionsachse p. Im Beispiel der Figur 1a respektive 1b, entspricht der Array A zur Modellierung der Retina 23 den Zellen eines Gitternetzes, das auf einer normal zur Projektionsachse p verlaufenden Ebene angeordnet ist, auf einer Höhe a, die dem Mittelpunkt der Ausdehnung der Retina 23 in Richtung der Projektionsachse p entspricht. In einer aufwendigeren Variante entspricht der Array A den Messpunkten eines auf die tatsächliche Oberfläche der betreffenden Augenstruktur gelegten Gitternetzes, d.h. die einzelnen Array Elemente haben eine unterschiedliche zugeordnete Höhe respektive Tiefe in Richtung der Projektionsachse p. Mehrere Augenstrukturen respektive Oberflächen von Augenstrukturen sind jeweils durch einen separaten Array A modelliert. Neben der Retina 23 werden beispielsweise weitere Arrays A für die Cornea und Iris sowie die vordere und hintere Linsenoberfläche definiert. In einer Ausführungsvariante ist ein dreidimensionaler Array vorgesehen, dessen Arrayelemente jeweils einem Volumenelement oder Voxel des Auges 2 mit einem zugeordneten Messpunkt entsprechen. Die Volumenelemente oder Voxel sind kubisch und weisen eine Kantenlänge von $10\mu m$-$500\mu m$ auf. In einer Variante wird ein dreidimensionales Augenmodell durch mehrere übereinanderliegende Gewebeschichten respektive entsprechende Arrays A definiert.

In einer Ausführungsvariante umfasst die Schutzvorrichtung 1 ein Messsystem 14, das eingerichtet ist, die Augendaten während der Behandlung des Auges 2 zu bestimmen, beispielsweise ein interferometrisches Messsystem, das optisch in den Strahlengang des optischen Therapiesystems 3 eingekoppelt ist. Der Prozessor 10 ist eingerichtet das Augenmodell 12 basierend auf den vom Messsystem 14 erfassten und gelieferten Augendaten zu erzeugen. In einer alternativen Ausführungsvariante umfasst die Schutzvorrichtung 1 ein Positionierungssystem 15, das eingerichtet ist, während der Behandlung eine Relativposition des Auges 2 zu bestimmen und ein auf vor der Behandlung erfassten Augendaten basierendes und erzeugtes Augenmodell abhängig von der Relativposition bezüglich dem optischen Therapiesystem 3 zu positionieren.

Das Steuerdatenmodul 11 ist eingerichtet, Steuerdaten zu erfassen, die Strahlparameter des Laserstrahls L, beispielsweise die Strahlleistung des Laserstrahls L, ein Scannmuster (zweidimensionales xy-Scannmuster oder dreidimensionales xyz-Scannmuster) für den Laserstrahl L und die Scanngeschwindigkeit des optischen Therapiesystems 3 definieren. Abhängig von der Ausführungsvariante umfassen die Strahlparameter des Laserstrahls L überdies Pulsbreite, Pulsrate, Pulsenergie, Pulsintensität, Fokusgrösse, Laserstrahlintensitätsprofil I(x), und Divergenz des Laserstrahls L. In einer so genannten Online-Konfiguration erfasst der Prozessor 10 die Steuerdaten während der Behandlung des Auges 2 vom optischen Therapiesystem 3 respektive vom Lasersystem 31 und/oder vom Scannersystem 32, beispielsweise über eine drahtlose oder drahtgebundene Kommunikationsschnittstelle. In einer Offline-Konfiguration erfasst der Prozessor 10 die Steuerdaten über eine Benutzerschnittstelle 13 von einem Benutzer, beispielsweise zur Simulation einer Behandlung des Auges 2.

Der Prozessor 10 ist eingerichtet, basierend auf den erfassten Augendaten und den erfassten oder eingegebenen Steuerdaten rechnerisch einen sich auf oder in einer Augenstruktur 23 bewegenden Lichtfleck S zu modellieren und dessen Bewegung zu simulieren. Die Modellierung und Simulation von Lichtflecken S wird insbesondere für Gewebeschichten angewendet, die eine hohe Absorption für die Lichtwellenlänge des Laserstrahls L aufweisen. Wie in den Figuren 1a, 1b illustriert ist, bestimmt der Prozessor 10 Umriss und Grösse des Lichtflecks S auf der durch den Array A modellierten Augenstruktur respektive dessen Oberfläche aufgrund des Strahlkegels C des Laserstrahls L respektive dessen Divergenzwinkel $\theta$ und der Distanz der betreffenden Augenstruktur zum Fokus F des Laserstrahls L. Dabei ist der Strahlkegel C respektive der Divergenzwinkel $\theta$ vom Brechungsindex n_b des sich nach dem Fokus F befindenden Mediums abhängig. Die Fokusposition z ist vom Brechungsindex n_a des Mediums abhängig, das sich vor dem Fokus F befindet. Vereinfachend werden für die Brechungsindizes n_a und n_b für die Medien gemittelte Werte angenommen. In einem Worst Case Ansatz werden Medien und Grenzflächen vorausgesetzt, die den tendenziell kleinsten Fleck auf der Retina erzeugen. Diese Zusammenhänge werden komplexer, wenn sich noch mehr Medien im System befinden und berücksichtigt werden sollen, zum Beispiel Flüssigkeit in einem mit Flüssigkeit gefüllten Patienteninterface, Cornea, Vorderkammerwasser, Linsenkörper, Glaskörper, etc. Sind Grenzflächen zwischen den Medien geneigt, werden die den Kegel bildenden Strahlen zusätzlich seitlich abgelenkt. All das wird durch das Augenmodel modelliert, das ggf. auch noch den Bereich zwischen Strahlaustritt am Lichtprojektor 33 des optischen Therapiesystems 3 und dem Auge 2 erfasst. Dazu werden in einer einfachen Form, die Brechung der Achse des Strahlkegels sowie die Änderung des Kegelwinkels mit Hilfe des Snelliusschen Brechungsgesetz an jeder Grenzfläche bestimmt. Bei vom Kegel abweichenden Formen und grösseren Grenzwinkeln werden mehrere Strahlen des Strahlenbündels mit Ray-Tracing-Methoden berechnet und damit eine verbesserte Abbildung erzielt.

Überdies bestimmt der Prozessor 10 basierend auf dem Intensitätsprofil I(x) des Laserstrahls L, beispielsweise ein Gauss'sches Intensitätsprofil im Laserquerschnitt eines Gauss-Strahls, das Intensitätsprofil des durch den Strahlkegel C definierten Lichtflecks S. Wie auf der rechten Seite der Figur 1b anhand des vergrössert abgebildeten Array-Ausschnitts A' dargestellt ist, wird das Intensitätsprofil I(x) des Lichtflecks S beispielsweise entsprechend der Auflösung des Arrays A auf die Augenstruktur abgebildet. Bei einer Bewegung des Laserstrahls L und damit einhergehend des Lichtflecks S werden die durch die Array-Elemente Axy repräsentierten Flächenelemente der Augenstruktur mit unterschiedlichen und sich mit der Bewegung ändernden Intensitätswerten bestrahlt.

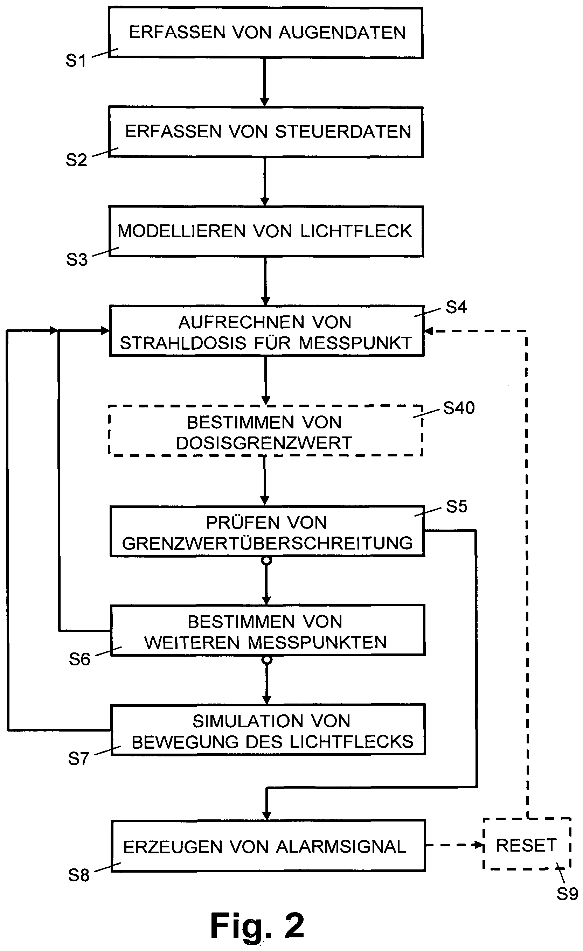

Nachfolgend werden die durch den Prozessor 10 durchgeführten Schritte zum Schützen von Gewebe bei der Behandlung des Auges 2 mit dem vom Lasersystem 31 erzeugten und durch das Scannersystem 32 abgelenkten Laserstrahl L an einer beispielhaften Sequenz mit Bezug zur Figur 2 beschrieben.

Im Schritt S1, erfasst der Prozessor 10 die Augendaten des Patienten und erzeugt darauf basierend wie oben beschrieben ein entsprechendes Augenmodell, das einen oder mehrere Arrays A umfasst.

Im Schritt S2, erfasst der Prozessor 10 wie oben beschrieben die Steuerdaten, welche die Strahlparameter des Laserstrahls L und das Scannmuster für den Laserstrahl L definieren. Im Schritt S3, modelliert der Prozessor 10 wie oben beschrieben den Lichtfleck S basierend auf den erfassten Steuerdaten und auf der oder den durch die Augendaten definierten Augenstruktur(en) respektive deren Oberfläche(n). Dabei wird der Austrittswinkel des Laserstrahls L am Austrittsfenster des Lichtprojektors 33 bezüglich der Projektionsachse p durch das Scannersystem 32 respektive das Scannmuster bestimmt. Der Strahlverlauf des Laserstrahls L respektive Richtungsänderungen des Laserstrahls L werden aufgrund der Brechungsindizes n_a und n_b der sich vor respektive nach dem Fokus F befindenden Medien bestimmt und modelliert. Der Fachmann wird verstehen, dass in der Modellierung und Simulation des Strahlverlaufs sowie dem Strahlkegel C und sich daraus ergebenden Lichtflecken S weitere Strukturen respektive Medien im Strahlengang mit ihren entsprechenden Brechungsindizes und Brechwerten berücksichtigt werden können.

Im Schritt S4, erfasst der Prozessor 10 für die durch den Lichtfleck S abgedeckten respektive bestrahlten Flächenelemente der Augenstruktur die Strahldosis. Dabei wird jeweils abhängig vom Intensitätsprofil I(x) des Lichtflecks S beim betreffenden Flächenelement die durch den Laserstrahl L abgegebene Strahldosis im Array-Element Axy, das dem betreffenden Flächenelement zugeordnet ist, aufaddiert. Die Strahl- oder Bestrahlungsdosis wird durch Integration der Strahlleistung des Laserstrahls L am betreffenden Flächenelement als Energiewert aufgerechnet. Jedes Array-Element Axy entspricht somit einem Messpunkt, der dem betreffenden Flächenelement der Augenstruktur zugeordnet ist.

In optionalen Schritt S40 führt der Prozessor 10 überdies eine akkumulierte Bestrahlungszeit t für den Messpunkt nach. Das heisst der Prozessor 10 ermittelt für den Messpunkt eine Bestrahlungszeitdauer t, während der das betreffende Flächenelement der Augenstruktur durch den modellierten Lichtfleck S bestrahlt wird. Der Prozessor 10 berechnet zudem für den Messpunkt respektive das Flächenelement einen sich ändernden Dosisgrenzwert $D_{max}$ abhängig von der Bestrahlungszeitdauer t am betreffenden Messpunkt respektive Flächenelement. In einer Ausführungsvariante berechnet der Prozessor 10 den Dosisgrenzwert $D_{max}$ für die Messpunkte überdies abhängig von der Wellenlänge des Laserstrahls L. Der Prozessor 10 berechnet den Dosisgrenzwert $D_{max}$ für die Messpunkte beispielsweise gemäss der nachfolgenden Formel:

$$D_{max} = C \cdot t^{\frac{3}{4}},$$

wobei C eine von der Wellenlänge des Laserstrahls L abhängige Konstante und t die Bestrahlungszeitdauer am betreffenden Messpunkt sind.

Im Schritt S5, überprüft der Prozessor 10, ob die Strahldosis, die im Schritt S4 für den durch das Array-Element Axy repräsentierten Messpunkt aufaddiert wurde, den definierten Dosisgrenzwert $D_{max}$ überschreitet. Falls der Dosisgrenzwert $D_{max}$ tatsächlich überschritten wird, fährt der Prozessor 10 im Schritt S8 mit der Erzeugung eines Notsignals fort. Andernfalls, bestimmt der Prozessor 10 im Schritt S6, ob weitere Messpunkte zu bearbeiten sind, die vom aktuellen Lichtfleck S bestrahlt werden. Das Erzeugen eines Alarmsignals kann je nach Art des Dosiswerts entweder zu einem Abbruch der Behandlung oder in einer weiteren Ausführungsform zu deren Unterbruch führen. Wie mit dem optionalen Schritt S9 schematisch dargestellt ist, wird bei einem Unterbruch die Behandlung nach einer bestimmten Wartezeit durch Zurücksetzen ("Reset") der akkumulierten Dosiswerte wieder fortgeführt. Ein typischer Dosisgrenzwert $D_{max}$ ermittelt sich zum Beispiel bei Ultrakurzpuls Infrarotlasern mit obenstehender Gleichung mit C=10 J/cm² und t in Sekunden; genauere Werte lassen sich z.B. mit Normen wie der ANSI Z136.1 oder der ISO 15004 bestimmen.

Wenn weitere (durch den Lichtfleck S abgedeckte Flächenelemente und entsprechende) Messpunkte zu berechnen sind, fährt der Prozessor damit im Schritt S4 fort. Andernfalls fährt der Prozessor 10 im Schritt S7 mit der Simulation der Bewegung des Lichtflecks S fort.

Im Schritt S7, simuliert der Prozessor 10 die Bewegung des Lichtflecks S aufgrund der durch die Steuerdaten definierten Scannmuster und Scanngeschwindigkeit. Dazu simuliert der Prozessor 10 den abgelenkten Laserstrahl L gemäss dem durch die Steuerdaten definierten Scannmuster. Das heisst der Prozessor 10 bestimmt die Ausrichtung und den Strahlenverlauf des Laserstrahls L ausgehend vom Austrittsfenster am Lichtprojektor 33 durch das durch die Augendaten des Augenmodells 12 bestimmte Auge 2 respektive dessen Augenstrukturen abhängig von der Ablenkung des Laserstrahls L, die durch das Scannersystem 32 entsprechend dem durch die Steuerdaten definierten Scannmuster und die Brechungsindizes n_a und n_b der sich vor respektive nach dem Fokus F befindenden Medien bewirkt wird, wie obenstehend im Zusammenhang mit Schritt S3 beschrieben wurde. Der Fachmann wird verstehen, dass für die Simulation der Ablenkung des Laserstrahls L und der damit verbundenen Bewegung des Lichtflecks S sowie der durch den bewegten Lichtfleck S bewirkten Bestrahlung von Flächenelementen der Augenstrukturen unterschiedliche Zeitbasiswerte $t_{sim}$ für aufeinanderfolgende Simulationsschritte wählbar sind, die abhängig von der Ablenkungs- respektive Scanngeschwindigkeit des optischen Therapiesystems 3 und andererseits von der gewünschten Auflösung respektive Grösse der Flächenelemente gewählt werden. In einer Ausführungsvariante entspricht der Zeitbasiswert $t_{base}$ eines Simulationsschritts der Zeitdauer, die der abgelenkte Laserstrahl L respektive der darauf basierte Lichtfleck S benötigt um die Länge respektive Breite $d_{pixel}$ eines Flächenelements zurückzulegen. Damit ergibt sich der Zeitbasiswert $t_{base}$ bei einer Scanngeschwindigkeit $v_{scan}$ nach

$$t_{base} = \frac{d_{pixel}}{v_{scan}}$$ . Wird der Zeitbasiswert $t_{base}$ grösser als $\frac{d_{laserstrahl}}{v_{scan}}$ gewählt (bei einem Laserstrahldurchmesser $d_{laserstrahl}$) werden einige Array-Elemente Axy auf der Bahn des Lichtflecks S nicht mehr beschrieben. In einem solchen Betriebsmodus wird in Kauf genommen, dass die Bestrahlungsdosis für die nicht oder ungenügend beschrieben Array-Elemente Axy auf die Elemente, die beim betreffenden Zeitschritt beleuchtet und im Array A erfasst werden, zusätzlich aufaddiert wird. Diese Art der Unterabtastung entspricht einer pessimistischen Berechnung der Bestrahlungsdosiswerte und ist damit unkritisch, auch wenn dadurch der maximal erlaubte Dosisgrenzwert $D_{max}$ in der Behandlung nicht tatsächlich erreicht werden kann. Zur Erzielung einer realitätsnäheren Verteilung der Bestrahlungsdosis wird die Verteilung der Strahlintensität des Laserstrahls L mit dem Bewegungsvektor des Lichtflecks S gefaltet und die derart verzerrte Verteilung in die Array-Elemente Axy geschrieben. Dabei definiert der Bewegungsvektor die Bewegung des Lichtflecks S und entspricht der Verschiebung von entsprechenden Punkten des Lichtflecks S, z.B. das Zentrum der Lichtverteilung, während eines Zeitschritts. Typische Werte für die Zeitbasiswerte $t_{base}$ liegen im ms bzw. μs Bereich, wenn Scanngeschwindikeiten $v_{scan}$ im Bereich von 0.1 bis 10 m/s vorliegen. Basierend auf der neuen Position des bewegten Lichtflecks S aktualisiert der Prozessor 10 im Schritt S4 für die durch den Lichtfleck S abgedeckten respektive bestrahlten Flächenelemente der Augenstruktur die Strahldosis. Das heisst für die den betreffenden Flächenelementen zugeordneten Array-Elemente Axy wird die vom Intensitätsprofil I(x) des Lichtflecks S an den betreffenden Stellen abhängige Strahlleistung des Laserstrahls L ermittelt und darauf basierend die im Flächelement respektive am zugeordneten Messpunkt eingestrahlte Laserenergie berechnet und als Strahldosis dem betreffenden Array-Element Axy hinzuaddiert.

Wenn die akkumulierte Strahldosis eines Array-Elements Axy den Dosisgrenzwert $D_{max}$ überschreitet, erzeugt der Prozessor 10 im Schritt S8 ein Notsignal. In der Online-Konfiguration, in der die Erfassung der mit dem Lichtfleck S simulierten Strahlbelastung (in Echtzeit) während und mit der realen Behandlung des Auges 2 erfolgt, leitet der Prozessor 10 das Notsignal zum Unterbrechen der Behandlung an das optische Therapiesystem 3 respektive Lasersystem 31 und/oder Scannersystem 32, beispielsweise durch Stoppen der Erzeugung der Laserpulse und/oder Schliessen einer Blende zum Blockieren der Laserpulse. Mit anderen Worten, in der Online-Konfiguration erfolgt die Bestimmung der simulierten Strahlbelastung parallel zu und synchronisiert mit der realen, tatsächlich stattfindenden Ausführung der Augenbehandlung. In der Offline-Konfiguration, in der die Erfassung der mit dem Lichtfleck S simulierten Strahlbelastung zur Vorbereitung einer Behandlung des Auges 2 erfolgt, gibt der Prozessor 10 das Notsignal als Warnhinweis über die Benutzerschnittstelle 13 aus, beispielsweise als Warnhinweis auf einer Anzeige.

Abschliessend soll angeführt werden, dass in der Beschreibung zwar Computerprogrammcode spezifischen funktionalen Modulen zugeordnet wurde und dass die Ausführung von Schritten in einer bestimmten Reihenfolge dargestellt wurde, dass der Fachmann jedoch verstehen wird, dass der Computerprogrammcode unterschiedlich strukturiert und die Reihenfolge von mindestens gewissen Schritten geändert werden kann, ohne dabei vom Schutzgegenstand abzuweichen.

**Patentansprüche**

1. Vorrichtung (1) zum Schützen von Gewebe bei der Behandlung eines Auges (2) mit einem von einem optischen Therapiesystem (3) erzeugten und durch ein Scannersystem (32) abgelenkten Laserstrahl (L), umfassend:

   ein Augenmodell (12), das Augendaten umfasst, welche Abmessungen und Lage von Augenstrukturen definieren,
   ein Steuerdatenmodul (11), das eingerichtet ist, Steuerdaten zu erfassen, welche Strahlparameter des Laserstrahls (L) und ein Scannmuster für den Laserstrahl (L) definieren,
   **gekennzeichnet durch** einen Prozessor (10), der eingerichtet ist, basierend auf den Augendaten und den Steuerdaten rechnerisch einen sich auf oder in einer Augenstruktur bewegenden Lichtfleck (S) zu simulieren, für mehrere Messpunkte (Axy) der Augenstruktur laufend mit dem sich bewegenden Lichtfleck (S) eine am betreffenden Messpunkt (Axy) durch den sich bewegenden Lichtfleck (S) abgegebene Strahldosis aufzurechnen, und beim Überschreiten eines Dosisgrenzwerts $D_{max}$ an einem der Messpunkte (Axy) ein Notsignal zu erzeugen.

2. Vorrichtung (1) nach Anspruch 1, **dadurch gekennzeichnet, dass** die Steuerdaten eine Scanngeschwindigkeit für das optische Therapiesystem (3) definieren, und dass der Prozessor (10) eingerichtet ist, den sich bewegenden Lichtfleck (S) mit einer auf der Scanngeschwindigkeit basierenden Bewegungsgeschwindigkeit zu simulieren.

3. Vorrichtung (1) nach einem der Ansprüche 1 oder 2, **dadurch gekennzeichnet, dass** die Strahlparameter die Strahlleistung des Laserstrahls (L) umfassen, und dass der Prozessor (10) eingerichtet ist, die Strahldosis an den Messpunkten (Axy) jeweils als Energiewert durch Integration der Strahlleistung des Laserstrahls (L) aus dem sich über den betreffenden Messpunkt (Axy) bewegenden Lichtfleck (S) aufzurechnen.

4. Vorrichtung (1) nach einem der Ansprüche 1 bis 3, **dadurch gekennzeichnet, dass** der Prozessor (10) eingerichtet ist, für die Messpunkte (Axy) der Augenstruktur laufend eine sich durch den bewegenden Lichtfleck (S) ergebende Bestrahlungszeitdauer zu ermitteln, und den Dosisgrenzwert $D_{max}$ für die Messpunkte (Axy) jeweils abhängig von der Bestrahlungszeitdauer am betreffenden Messpunkt (Axy) zu definieren.

5. Vorrichtung (1) nach einem der Ansprüche 1 bis 4, **dadurch gekennzeichnet, dass** der Prozessor (10) eingerichtet ist, den Dosisgrenzwert $D_{max}$ für die Messpunkte (Axy) abhängig von der Wellenlänge des Laserstrahls (L) zu definieren.

6. Vorrichtung (1) nach einem der Ansprüche 1 bis 5, **dadurch gekennzeichnet, dass** der Prozessor (10) eingerichtet ist, den Dosisgrenzwert $D_{max}$ für die Messpunkte (Axy) abhängig von einer Bestrahlungszeitdauer $t$ am betreffenden Messpunkt (Axy) zu bestimmen.

7. Vorrichtung (1) nach einem der Ansprüche 1 bis 6, **dadurch gekennzeichnet, dass** der Prozessor (10) eingerichtet ist, den Dosisgrenzwert $D_{max}$ für die Messpunkte (Axy) jeweils gemäss der Formel $D_{max} = C \cdot t^{\frac{3}{4}}$ zu berechnen, wobei $C$ eine von Strahlparametern des Laserstrahls (L) abhängige Konstante und $t$ die Bestrahlungszeitdauer am betreffenden Messpunkt (Axy) sind.

8. Vorrichtung (1) nach einem der Ansprüche 1 bis 7, **dadurch gekennzeichnet, dass** der Prozessor (10) eingerichtet ist, den abgelenkten Laserstrahl (L) basierend auf dem durch die Steuerdaten definierten Scannmuster zu simulieren, die Augenstruktur basierend auf den Augendaten zu modellieren, und den sich bewegenden Lichtfleck (S) basierend auf dem simulierten abgelenkten Laserstrahl (L) und der modellierten Augenstruktur zu simulieren.

9. Vorrichtung (1) nach einem der Ansprüche 1 bis 8, **dadurch gekennzeichnet, dass** der Prozessor (10) eingerichtet ist, den sich bewegenden Lichtfleck (S) auf einer Oberfläche der Augenstruktur zu ermitteln, und die sich durch den bewegenden Lichtfleck (S) ergebende Strahldosis für mehrere Messpunkte (Axy) auf der Oberfläche der Augenstruktur aufzurechnen.

10. Vorrichtung (1) nach einem der Ansprüche 1 bis 9, **dadurch gekennzeichnet, dass** der Prozessor (10) eingerichtet ist, den sich bewegenden Lichtfleck (S) und die Messpunkte (Axy) auf einer Oberfläche einer oder mehrerer Augenstrukturen (23) aus der folgenden Liste zu bestimmen: Epithel, Endothel, Iris, Sklera, vordere Linsenoberfläche, hintere Linsenoberfläche und Netzhautoberfläche.

11. Vorrichtung (1) nach einem der Ansprüche 1 bis 10, **dadurch gekennzeichnet, dass** die Vorrichtung (1) mit dem optischen Therapiesystem (3) und dem Scannersystem (32) verbunden ist, und dass der Prozessor (10) eingerichtet ist, die Steuerdaten während der Behandlung des Auges (2) vom optischen Therapiesystem (3) und vom Scannersystem (32) zu erfassen, und das Notsignal zum Unterbrechen der Behandlung an das optische Therapiesystem (3) zu übermitteln.

12. Vorrichtung (1) nach einem der Ansprüche 1 bis 11, **dadurch gekennzeichnet, dass** der Prozessor (10) eingerichtet ist, die Steuerdaten zur Simulation der Behandlung des Auges (2) über eine Benutzerschnittstelle (13) zu erfassen, und das Notsignal als Warnhinweis über die Benutzerschnittstelle (13) auszugeben.

13. Vorrichtung (1) nach einem der Ansprüche 1 bis 12, **dadurch gekennzeichnet, dass** die Steuerdaten einen oder mehrere Strahlparameter aus der folgenden Liste definieren: Pulsbreite, Pulsrate, Pulsenergie, Pulsintensität, Fokusgrösse, Laserstrahlintensitätsprofil, und Divergenz des zur fokussierten Projektion vorgesehenen Laserstrahls (L), und dass der Prozessor (10) eingerichtet ist, den sich bewegenden Lichtfleck (S) basierend auf dem einen oder mehreren Strahlparametern zu simulieren.

14. Vorrichtung (1) nach einem der Ansprüche 1 bis 13, **gekennzeichnet durch** ein Messsystem (14), das eingerichtet ist, die Augendaten während der Behandlung des Auges (2) zu bestimmen.

**15.** Vorrichtung (1) nach einem der Ansprüche 1 bis 14, **gekennzeichnet durch** ein Positionierungssystem (15), das eingerichtet ist, während der Behandlung eine Relativposition des Auges (2) zu bestimmen und das Augenmodell abhängig von der Relativposition bezüglich dem optischen Therapiesystem (3) zu positionieren.

## Claims

**1.** Device (1) for protecting tissue when treating an eye (2) using a laser beam (L) generated by an optical therapy system (3) and deflected by a scanner system (32), including:

an eye model (12) including eye data which define dimensions and locations of eye structures, a control data module (11) configured to register control data which define beam parameters of the laser beam (L) and a scanning pattern for the laser beam (L), **characterized by** a processor (10) configured to simulate by computation a light spot (S) moving on or in an eye structure on the basis of the eye data and the control data, to add up, for a plurality of measurement points (Axy) of the eye structure and continuously with the moving light spot (S), a beam dose emitted at the relevant measurement point (Axy) by the moving light spot (S) and to generate an emergency signal if a dose limit $D_{max}$ is exceeded at one of the measurement points (Axy).

**2.** Device (1) according to Claim 1, **characterized in that** the control data define a scanning speed for the optical therapy system (3) and **in that** the processor (10) is configured to simulate the moving light spot (S) with a movement speed based on the scanning speed.

**3.** Device (1) according to either of Claims 1 and 2, **characterized in that** the beam parameters include the beam power of the laser beam (L) and **in that** the processor (10) is configured to add up the beam dose at the measurement points (Axy), respectively as an energy value, by integrating the beam power of the laser beam (L) from the light spot (S) moving over the relevant measurement point (Axy).

**4.** Device (1) according to one of Claims 1 to 3, **characterized in that** the processor (10) is configured to continuously establish, for the measurement points (Axy) of the eye structure, an irradiation time resulting from the moving light spot (S) and to define the dose limit $D_{max}$ for the measurement points (Axy) in a manner dependent on the irradiation time at the respective measurement point (Axy) concerned.

**5.** Device (1) according to one of Claims 1 to 4, **characterized in that** the processor (10) is configured to define the dose limit $D_{max}$ for the measurement points (Axy) in a manner dependent on the wavelength of the laser beam (L).

**6.** Device (1) according to one of Claims 1 to 5, **characterized in that** the processor (10) is configured to determine the dose limit $D_{max}$ for the measurement points (Axy) in a manner dependent on an irradiation time t at the relevant measurement point (Axy).

**7.** Device (1) according to one of Claims 1 to 6, **characterized in that** the processor (10) is configured to calculate the respective dose limit $D_{max}$ for the measurement points (Axy) according to the equation

$$D_{max} = C \cdot t^{\frac{3}{4}},$$ where $C$ is a constant dependent on beam parameters of the laser beam (L) and $t$ is the irradiation time at the relevant measurement point (Axy).

**8.** Device (1) according to one of Claims 1 to 7, **characterized in that** the processor (10) is configured to simulate the deflected laser beam (L) on the basis of the scanning pattern defined by the control data, to model the eye structure on the basis of the eye data, and to simulate the moving light spot (S) on the basis of the simulated deflected laser beam (L) and the modeled eye structure.

**9.** Device (1) according to one of Claims 1 to 8, **characterized in that** the processor (10) is configured to determine the moving light spot (S) on a surface of the eye structure and to add up the beam dose resulting from the moving light spot (S) for a plurality of measurement points (Axy) on the surface of the eye structure.

**10.** Device (1) according to one of Claims 1 to 9, **characterized in that** the processor (10) is configured to determine the moving light spot (S) and the measurement points (Axy) on a surface of one or more eye structures (23) from the following list: epithelium, endothelium, iris, sclera, front lens surface, rear lens surface and retinal surface.

**11.** Device (1) according to one of Claims 1 to 10, **characterized in that** the device (1) is connected to the optical therapy system (3) and the scanner system (32) and **in that** the processor (10) is configured to register the control data from the optical therapy system (3) and from the scanning system (32) during the treatment of the eye (2) and to transmit the emergency signal for interrupting the treatment to the optical therapy system (3) .

**12.** Device (1) according to one of Claims 1 to 11, **characterized in that** the processor (10) is configured to register the control data for simulating the treatment of the eye (2) by way of a user interface (13) and to output the emergency signal as a warning notification by way of the user interface (13).

**13.** Device (1) according to one of Claims 1 to 12, **characterized in that** the control data define one or more beam parameters from the following list: pulse width, pulse rate, pulse energy, pulse intensity, focal size, laser beam intensity profile and divergence of the laser beam (L) provided for the focussed projection, and **in that** the processor (10) is configured to simulate the moving light spot (S) on the basis of the one or more beam parameters.

**14.** Device (1) according to one of Claims 1 to 13, **characterized by** a measurement system (14) configured to determine the eye data during the treatment of the eye (2) .

**15.** Device (1) according to one of Claims 1 to 14, **characterized by** a positioning system (15) configured to determine a relative position of the eye (2) during the treatment and to position the eye model relative to the optical therapy system (3) in a manner dependent on the relative position.

**Revendications**

**1.** Dispositif (1) de protection de tissus pendant le traitement d'un œil (2) avec un faisceau laser (L) généré par un système de thérapie optique (3) et dévié par un système de scanner (32), ledit dispositif comprenant :

un modèle oculaire (12) qui comprend des données oculaires qui définissent les dimensions et la position de structures oculaires,
un module de données de commande (11) qui est conçu pour acquérir des données de commande qui définissent des paramètres de faisceau du faisceau laser (L) et un motif de balayage pour le faisceau laser (L),
**caractérisé par** un processeur (10) qui est conçu pour simuler, sur la base des données oculaires et des données de commande, un point lumineux (S) mobile sur ou dans une structure oculaire, calculer pour plusieurs points de mesure (Axy) de la structure oculaire en continu avec le point lumineux mobile (S) une dose de rayonnement émise par le point lumineux mobile (S) au point de mesure correspondant (Axy), et générer un signal d'urgence lorsqu'une limite de dose $D_{max}$ est dépassée à un des points de mesure (Axy).

**2.** Dispositif (1) selon la revendication 1, **caractérisé en ce que** les données de commande définissent une vitesse de balayage pour le système de thérapie optique (3) et **en ce que** le processeur (10) est conçu pour simuler le point lumineux mobile (S) avec une vitesse de déplacement en fonction de la vitesse de balayage.

**3.** Dispositif (1) selon l'une des revendications 1 ou 2, **caractérisé en ce que** les paramètres de faisceau du faisceau comprennent la puissance de faisceau du faisceau laser (L) et **en ce que** le processeur (10) est conçu pour additionner la dose de faisceau aux points de mesure (Axy) en tant que valeur énergétique par intégration de la puissance de faisceau du faisceau laser (L) à partir du point lumineux (S) mobile sur le point de mesure correspondant (Axy).

**4.** Dispositif (1) selon l'une des revendications 1 à 3, **caractérisé en ce que** le processeur (10) est conçu pour déterminer pour les points de mesure (Axy) de la structure oculaire en continu une durée d'irradiation due au point lumineux mobile (S) et définir, respectivement, la limite de dose $D_{max}$ pour les points de mesure (Axy) en fonction de la durée d'irradiation au point de mesure correspondant (Axy).

**5.** Dispositif (1) selon l'une des revendications 1 à 4, **caractérisé en ce que** le processeur (10) est conçu pour définir la limite de dose $D_{max}$ pour les points de mesure (Axy) en fonction de la longueur d'onde du faisceau laser (L).

**6.** Dispositif (1) selon l'une des revendications 1 à 5, **caractérisé en ce que** le processeur (10) est configuré pour déterminer la valeur limite de dose $D_{max}$ pour les points de mesure (Axy) en fonction d'une période d'irradiation t au point de mesure concerné (Axy) .

**7.** Dispositif (1) selon l'une des revendications 1 à 6, **caractérisé en ce que** le processeur (10) est conçu pour calculer la limite de dose $D_{max}$ pour les points de mesure (Axy) à chaque fois selon la formule

$$D_{max} = C \cdot t^{\frac{3}{4}}$$ où C est une constante dépendante de paramètres de faisceau du faisceau laser (L) et $t$ est le temps d'irradiation au point de mesure correspondant (Axy).

**8.** Dispositif (1) selon l'une des revendications 1 à 7, **caractérisé en ce que** le processeur (10) est conçu pour simuler le faisceau laser dévié (L) sur la base du motif de balayage défini par les données de commande, modéliser la structure oculaire sur la base des données oculaires et simuler le point lumineux mobile (S) sur la base du faisceau laser dévié simulé (L) et de la structure oculaire modélisée.

9. Dispositif (1) selon l'une des revendications 1 à 8, **caractérisé en ce que** le processeur (10) est conçu pour déterminer le point lumineux mobile (S) sur une surface de la structure oculaire et calculer la dose de rayonnement, due au point lumineux mobile (S), pour plusieurs points de mesure (Axy) à la surface de la structure oculaire.

10. Dispositif (1) selon l'une des revendications 1 à 9, **caractérisé en ce que** le processeur (10) est conçu pour déterminer le point lumineux mobile (S) et les points de mesure (Axy) sur une surface d'au moins une structure oculaire (23) à partir de la liste suivante :
épithélium, endothélium, iris, sclérotique, surface antérieure du cristallin, surface postérieure du cristallin et surface rétinienne.

11. Dispositif (1) selon l'une des revendications 1 à 10, **caractérisé en ce que** le dispositif (1) est relié au système de thérapie optique (3) et au système de scanner (32) et **en ce que** le processeur (10) est conçu pour acquérir les données de commande pendant le traitement de l'œil (2) à partir du système de thérapie optique (3) et du système de scanner (32), et transmettre le signal d'urgence au système de thérapie optique (3) afin d'interrompre le traitement.

12. Dispositif (1) selon l'une des revendications 1 à 11, **caractérisé en ce que** le processeur (10) est conçu pour acquérir les données de commande par le biais d'une interface utilisateur (13) afin de simuler le traitement de l'œil (2) et délivrer le signal d'urgence sous la forme d'un avertissement par le biais de l'interface utilisateur (13).

13. Dispositif (1) selon l'une des revendications 1 à 12, **caractérisé en ce que** les données de commande définissent au moins un paramètre de faisceau à partir de la liste suivante : largeur d'impulsion, fréquence d'impulsion, énergie d'impulsion, intensité d'impulsion, dimension de foyer, profil d'intensité du faisceau laser et divergence du faisceau laser (L) prévu pour la projection focalisée et **en ce que** le processeur (10) est conçu pour simuler le point lumineux mobile (S) sur la base d'au moins un paramètre de faisceau.

14. Dispositif (1) selon l'une des revendications 1 à 13, **caractérisé par** un système de mesure (14) qui est conçu pour déterminer les données oculaires lors du traitement de l'œil (2).

15. Dispositif (1) selon l'une des revendications 1 à 14, **caractérisé par** un système de positionnement (15) qui est conçu pour déterminer une position relative de l'œil (2) pendant le traitement et positionner le modèle de l'œil en fonction de la position relative par rapport au système de thérapie optique (3).

EP 3 023 082 B1

**Fig. 1a**

**Fig. 1b**

12

S1 ERFASSEN VON AUGENDATEN

S2 ERFASSEN VON STEUERDATEN

S3 MODELLIEREN VON LICHTFLECK

S4 AUFRECHNEN VON STRAHLDOSIS FÜR MESSPUNKT

S40 BESTIMMEN VON DOSISGRENZWERT

S5 PRÜFEN VON GRENZWERTÜBERSCHREITUNG

S6 BESTIMMEN VON WEITEREN MESSPUNKTEN

S7 SIMULATION VON BEWEGUNG DES LICHTFLECKS

S8 ERZEUGEN VON ALARMSIGNAL

S9 RESET

**Fig. 2**

**IN DER BESCHREIBUNG AUFGEFÜHRTE DOKUMENTE**

**In der Beschreibung aufgeführte Patentdokumente**

- US 20140276680 A
- EP 2705812 A